# EUROPÄISCHE PATENTANMELDUNG

(11) **EP 4 632 731 A1**
(43) Veröffentlichungstag der Anmeldung: **15.10.2025**
(21) Anmeldenummer: 24169919.8
(22) Anmeldetag: 12.04.2024
(51) Int. Cl.: G10L 13/027, G10L 25/48, G10L 25/30, G10L 13/04, G10L 15/22, G10L 15/26, A61B 5/00, A61B 6/00, G06F 3/16

(54) **MEDIZINGERÄT MIT SYNTHETISCHER SPRACHAUSGABE UND VERFAHREN ZUM TRAINIEREN**

(71) Anmelder: Siemens Healthineers AG, 91301 Forchheim (DE)
(72) Erfinder: Höcht, Philipp, 91207 Lauf (DE); Grodzki, David, 91058 Erlangen (DE); Schneider, Rainer, 91315 Höchstadt (DE); Stranjak, Armin, 91080 Uttenreuth (DE); Würfl, Tobias, 91054 Erlangen (DE); Zeller, Mario, 91054 Erlangen (DE); Janetschke, Luise, 91448 Emskirchen (DE); Möck, Thomas, 90443 Nürnberg (DE); Hellinger, Marion, 91080 Uttenreuth (DE); Schmidt, Michaela, 91080 Uttenreuth (DE); Zhao, Xue Feng, Shenzhen, 518054 (CN)
(74) Vertreter: Siemens Healthineers Patent Attorneys

(57) **Zusammenfassung**

Die Erfindung betrifft ein Medizingerät mit einem Sprachsynthesesystem sowie ein Verfahren zur Durchführung einer Behandlung oder Untersuchung mit dem Medizingerät. In einem Schritt des Verfahrens wird ein Patient für eine Untersuchung bzw. Behandlung relativ zu dem Medizingerät positioniert. Eine Sprachanweisung wird von dem Medizingerät mittels des Sprachsynthesesystems ausgegeben und ein Untersuchungsschritts bzw. Behandlungsschritts an dem Patienten mit dem Medizingerät ausgeführt.

## Beschreibung

*Unabhängig vom grammatikalischen Geschlecht eines bestimmten Begriffes sind Personen mit männlicher, weiblicher oder anderer Geschlechteridentität mit umfasst.*

Die Erfindung betrifft ein Medizingerät zur Behandlung oder Untersuchung eines Patienten mit synthetischer Sprachausgabe an den Patienten. Darüber hinaus betrifft die Erfindung ein Verfahren zum Trainieren des Medizingeräts.

Bei einer Behandlung oder Untersuchung eines Patienten mit einem Medizingerät ist üblicherweise eine Kommunikation mit dem Patienten erforderlich, zumindest sofern er bzw. sie bei Bewusssein ist. Dabei auftretende Probleme werden nachfolgend an dem Beispiel eines Magnetresonanztomographen erläutert.

Magnetresonanztomographen sind bildgebende Vorrichtungen, die zur Abbildung eines Untersuchungsobjektes Kernspins des Untersuchungsobjektes mit einem starken äußeren Magnetfeld ausrichten und durch ein magnetisches Wechselfeld zur Präzession um diese Ausrichtung anregen. Die Präzession bzw. Rückkehr der Spins aus diesem angeregten in einen Zustand mit geringerer Energie wiederum erzeugt als Antwort ein magnetisches Wechselfeld, das über Antennen empfangen wird.

Mit Hilfe von magnetischen Gradientenfeldern wird den Signalen eine Ortskodierung aufgeprägt, die nachfolgend eine Zuordnung von dem empfangenen Signal zu einem Volumenelement ermöglicht. Das empfangene Signal wird dann ausgewertet und eine dreidimensionale bildgebende Darstellung des Untersuchungsobjektes bereitgestellt. Zum Empfang des Signals werden vorzugsweise lokale Empfangsantennen, sogenannte Lokalspulen verwendet, die zur Erzielung eines besseren Signal-Rauschabstandes unmittelbar am Untersuchungsobjekt angeordnet werden.

Die Untersuchung dauert oft etliche Minuten, und ist für den Patienten mit Enge im Patiententunnel und der Geräuschentwicklung der Gradientenspulen verbunden. Auch ist die Magnetresonanzabbildung empfindlich auf Bewegungen, sodass für ein optimales Ergebnis der Patient z.B. zeitweise den Atem anhalten muss. Es ist daher erforderlich, mit dem Patienten zu kommunizieren, und sei es auch nur, um ihn zu beruhigen.

Es sind aus diesem Grund unterschiedliche Kommunikationssysteme bekannt, die es einer Bedienperson erlauben, dem Patienten Hinweise und Anweisungen an den Patienten durch direkte Übertragung von Sprache in Echtzeit zu übermitteln. Die Verständlichkeit ist dabei auch sprecherabhängig.

Ähnliche Probleme existieren auch bei anderen Medizingeräten zur Untersuchung oder Behandlung eines Patienten wie beispielsweise Computertomographen oder PET-Systemen, aber auch therapeutischen Systemen wie Bestrahlungsvorrichtungen.

Es ist daher die Aufgabe der Erfindung, bei derartigen Medizingeräten eine Kommunikation mit dem Patienten zu verbessern.

Diese Aufgabe wird durch ein erfindungsgemäßes Medizingerät nach Anspruch 1, Ein Verfahren zur Durchführung einer Untersuchung bzw. Behandlung nach Anspruch 8 und ein Verfahren zum Trainieren des Medizingeräts nach Anspruch 12 gelöst.

Das erfindungsgemäße Medizingerät kann ein Magnetresonanztomograph, aber auch ein anderes bildgebendes oder therapeutisches Medizingerät wie beispielsweise ein Computertomograph, ein PET-System oder Bestrahlungsgeräte sein.

Das Medizingerät weist ein Sprachsynthesesystem auf. Unter Sprachsynthesesystem ist gemäß der Erfindung ein System zu verstehen, das aus einem Text eine akustische Ausgabe in einer natürlichen Sprache erzeugt. Ein Text kann beispielsweise eine Datei oder ein Datenstrom mit Zeichen in ASCII-Format sein, nicht jedoch eine elektronische Aufzeichnung akustischer Sprache in Form der Töne.

Es ist aber auch denkbar, dass das SprachsynthesesystemTeil einer Speech-to-Speech Umsetzung ist, also eine akustische Eingabe in Sprachform wiederum in eine akustische Ausgabe in Sprachform umgesetzt wird. Das kann wie nachfolgend erläutert über eine Umsetzung in Textform und daraus wieder eine Sprachausgabe synthetisiert wird. Es ist aber auch denkbar, dass eine Umsetzung nicht inhaltlich, sondern nur in Stimmfarbe, Betonung und den darin ausgedrückten Emotionen erfolgt. Dabei ist es denkbar, dass auch andere Metadaten anstelle der Texform zwischen der Spracheingabe und der Sprachausgabe genutzt werden. Denkbar wären beispielsweise unterschiedliche Formen einer Repräsentation von Lauten oder Phonemen. Es ist ebenso möglich, dass die Speech-to-Speech-Umsetzung mittels eines neuronalen Netzes erfolgt, sodass die Sprachsyntheseeinheit als eine oder mehrere Schichten in der Ausgabe des neuronalen Netzes realisiert ist. Die Repäsentation der Sprachinformation kann hier verschiedenste Formen annehmen. Die Funktion des erfindungsgemäßen Sprachsynthesesystems unterscheidet sich jedoch von klassischen Signalbearbeitungsfunktionen wie beispielsweise Frequenzfilterung, Frequenzumsetzung oder Dynamikkompression.

Unter "weist auf" ist dabei auch die Möglichkeit zu verstehen, dass das Sprachsynthesesystem funktional in das Magnetresonanzsystem eingebunden ist, aber räumlich getrennt ist. Das Sprachsysnthesesystem kann integraler Bestandtei des Medizingeräts sein, beispielsweise in dessen Gehäuse untergebracht sein, aber auch über eine Datenfernverbindung wie ein IP-Netzwerk und zumindest teilweise räumlich abgesetzt realisiert sein, beispielsweise in einem Datenzentrum oder einer Cloud. Das Sprachsynthesesystem kann auch funktional und räumlich aufgeteilt sein, beispielsweise die Umsetzung des Textes in eine lautbasierte Form wie z.B. in Phoneme in einer Cloud und die Umwandlung und Wiedergabe der Phoneme in akustischer Form in dem Medizingerät.

Die akustische Ausgabe ist für einen Patienten vorgesehen, um diesem Informationen wie z.B. Anweisungen zur vorzunehmenden Bilderfassung zu übermitteln. Die Ausgabe erfolgt akustisch in natürlicher Sprache. Als natürliche Sprache werden hier zur alltäglichen Kommunikation zwischen Menschen Sprachformen bezeichnet, im Gegensatz z.B. zu formalen Sprachen wie Programmiersprachen. Die Ausgabe der natürliche Sprache kann beispielsweise über einen Lautsprecher oder Kopfhörer erfolgen, der nahe bei dem Patienten angeordnet ist, beispielsweise in einem Patientenunnel eines Magnetresonanzsystems. Denkbar ist aber auch ein abgesetzter Lautsprecher, dessen akustisches Ausgangssignal durch einen Schallleiter zu dem Patienten übertragen wird, um z.B. im Fall des Magnetresonanztomographen die Bilderfassung nicht durch elektrische Signale oder Magnetfelder zu stören.

Auf vorteilhafte Weise ermöglicht das Sprachsynthesesystem eine Sprachausgabe an den Patienten mit konstanter Sprachqualität und gleicher Stimme, auch wenn die Bedienperson des Medizingeräts wechseln sollte oder durch die Bedienung abgelenkt ist.

Das erfindungsgemäße Verfahren zur Durchführung einer Behandlung oder Untersuchung weist den Schritt auf, den Patienten relativ zu dem Medizingerät so zu positionieren, dass die Behandlung bzw. Untersuchung erfolgen kann. Für einen Computertomographen oder Magnetresonanztomographen kann es sich beispielsweise darum handeln, dass der Patient auf einem Patiententisch in die Gantry bzw. den Patiententunnel eingeführt wird. Bei Bestrahlungsgeräten oder Röntgengeräten heißt dies beispielsweise, den Patienten in den Strahlengang des Medizingeräts zu bringen.

In einem weiteren Schritt wird eine Behandlung bzw. Untersuchung des Patienten mit dem Medizingerät ausgeführt. Dieser Schritt kann in weitere Teilschritte unterteilt sein, beispielsweise bei einer Sequenz eines Magnetresonanztomographen. Auch eine Bestrahlung kann beispielsweise mit der Atmung synchronisiert sein und dadurch periodisch unterbrochen werden. Der Schritt bzw. die Teilschritte können dabei selbstständig von einer Steuerung ausgeführt werden, von der Steuerung auf Eingabe des Bedieners teilautonom ausgeführt werden oder manuell vom Bediener gesteuert werden.

In einem anderen Schritt gibt das Sprachsynthesesystem eine Information oder eine Anweisung in akustischer Sprachform an den Patienten aus. Die Information wird dem Sprachsynthesesystem vorzugsweise in Textform zugeführt. Der Text kann von einer Bedienperson eingegeben werden, auch über ein Spracheingabesystem, oder ausgewählt sein, oder wie nachfolgend zu den Unteransprüchen erläutert, von dem Medizingerät selbstständig ausgewählt werden. Die Sprachausgabe kann eine Anweisung an den Patienten sein, beispielsweise eine Anweisung, die Luft anzuhalten, oder eine Antwort auf eine Frage des Patienten.

Vorzugsweise sind dabei der Schritt der Ausgabe und die Schritte bzw. Teilschritte der Behandlung bzw. Untersuchung in vorbestimmter zeitlicher Relation zueinander, beispielsweise, um durch Anweisungen an den Patienten diesen für den nächsten Teilschritt vorzubereiten. Auch Informationen an den Patienten betreffen vorzugsweise den Ablauf der Behandlung bzw. Untersuchung und sind deshalb mit dieser synchronisiert.

Das erfindungsgemäße Verfahren zur Durchführung einer Behandlung bzw. Untersuchung teilt die Vorteile des erfindungsgemäßen Medizingeräts.

Das erfindungsgemäße Verfahren zum Bereitstellen von Trainingsdaten für ein Large-Language-Modell (LLM) dient der Anpassung bzw. dem Retrainieren eines solchen Modells, um es in dem neu entwickelten Medizingerät einzusetzen. Dazu wird ein Large-Language-Model wie zum Beispiel GPT ergänzt bzw. retrainiert, um die generierten Texte des LLM um Behandlungs- bzw. Untersuchungs-spezifische Kontexte zu ergänzen.

In einem Schritt des Verfahrens wird eine Behandlung oder Untersuchung an einem Patienten mit dem Medizingerät durchgeführt. Für einen Magnetresonanztomographen kann dies beispielsweise eine Bilderfassung mittels des Magnetresonanztomographen sein. Bei einem Bestrahlungsgerät kann dies das Durchführen einer Bestrahlung des Patienten sein.

In einem weiteren Schritt werden Sprachanweisungen erfasst, die von einer Bedienperson im Ablauf der Behandlung bzw. Untersuchung an den Patienten übermittelt werden. Dies kann in transkribierter Textform oder auch als akustische Aufnahme erfolgen.

In einem anderen Schritt werden Systemzustände des Medizingeräts erfasst. Im Fall eines Magnetresonanztomographen kann dies beispielsweise der Zeitpunkt innerhalb einer Bilderfassungssequenz oder im Rahmen der Vorbereitung bzw. Positionierung des Patienten sein. Dabei wird eine zeitliche Relation der erfassten Daten festgehalten, insbesondere welche Sprachanweisung zu welchem Zeitpunkt der Behandlung bzw. Bilderfassung gegeben wurden.

Die Schritte werden zur Erfassung einer Vielzahl unterschiedlicher Daten für das Training mit unterschiedlichen Patienten und/oder unterschiedlichen Behandlungen bzw. Bilderfassungen wiederholt.

Auf vorteilhafte Weise kann mit den so erfassten Daten die Sprachausgabe des Large-Language-Models an den Patienten durch ein damit durchgeführtes Training optimiert werden.

Ein erfindungsgemäßes Verfahren ist zum Training des Sprachsynthesesystems des Medizingeräts vorgesehen, um diesem unterschiedliche Sprecher bzw. Stimmen für die Ausgabe beizubringen. In einem Schritt des Verfahrens wird eine Sprachprobe einer Zielperson erfasst, deren Stimme für die Sprachsynthese genutzt werden soll. Dies kann beispielsweise durch Vorlesen eines Probetextes erfolgen, wobei der Sprecher aufgenommen wird, beispielsweise ein Mikrofon die Sprache aufnimmt und diese in digitalisierter Form als Datei gespeichert wird. Die Aufnahme kann an dem Medizingerät erfolgen, beispielsweise aber auch abgesetzt mit z.B. einem Mobiltelefon, wobei die gespeicherte Sprachdatei an das Medizingerät übertragen wird.

In einem weiteren Schritt wird das Sprachsynthesesystem mit den Sprachproben trainiert. Je nach Art des Sprachsynthesizers können dabei Phoneme oder andere Sprachelemente aus den Sprachproben extrahiert und für die Sprachsynthese abgelegt werden. Es ist aber auch denkbar, dass akustische Parameter der Stimme wie Spektren, Resonanzen und Anschwingzeiten erfasst werden und für eine Sprachsynthese auf Basis eines akustischen Modells erfolgt. Auch Sprachsynthese mittels eines neuronalen Netzwerks ist denkbar, wobei das neuronale Netz vorzugsweise mittels Backpropagation trainiert wird, möglichst dem Sprecher ähnliche Sprachelemente bzw. Phoneme zu erzeugen.

Auf vorteilhafte Weise ermöglicht das Verfahren zum Trainieren des Sprachsynthesizers, die Sprachausgaben schnell unterschiedlichen Sprechern anzupassen und so einem Patienten individuell möglichst gewohnte Klänge zu bieten.

Weitere vorteilhafte Ausführungsformen sind zu den Unteransprüchen angegeben.

In einer denkbaren Ausführungsform des erfindungsgemäßen Medizingeräts ist das Sprachsynthesesystem ausgebildet, unterschiedliche Stimmen für die Sprachausgabe zu erlernen und bei der Sprachausgabe zu nutzen. Denkbar ist es beispielsweise, dass das Sprachsynthesesystem eine Eingangsschnittstelle für akustische Daten aufweist. Das Sprachsynthesesystem ist ausgelegt, über diese Eingangsschnittstelle Sprachproben einer Person zu empfangen und diese zu nutzen, um Sprachmuster der Person zu erlernen und diese für künftige Sprachausgaben zu nutzen. Denkbar ist es beispielsweise, dass eine vertraute Person des Patienten Sprachproben durch Vorlesen eines Textes liefert, diese über ein Mikrofon von dem Sprachsynthesesystem erfasst und erlernt werden, sodass danach dem Patienten Anweisungen mit der vertrauten Stimme erfolgen können.

Auf vorteilhafte Weise ermöglicht es die Lernfähigkeit des Sprachsystems, dem Patienten Anweisungen mit vertrauter Stimme zu geben, was die Verständlichkeit erhöht und den Patienten beruhigt.

In einer möglichen Ausführungsform des erfindungsgemäßen Medizingeräts weist das Sprachsynthesesystem eine Textschnittstelle zur Eingabe der Sprachanweisungen auf. Über die Textschnittstelle kann ein auszugebender Text von einer oder mehreren Teilsystemen des Medizingeräts zugeführt werden. Beispielsweise kann über eine Tastatur eine Eingabe durch eine Bedienperson erfolgen, denkbar ist auch die Auswahl vorbestimmter Ausgaben über eine grafische Bedienoberfläche. Weitere Quellen für die Textschnittstelle sind nachfolgend zu den Unteransprüchen erläutert.

Die Textschnittstelle ermöglicht auf vorteilhafte Weise flexible Auswahl und Anpassung von Informationen für den Patienten, die über das Sprachsynthesesystem ausgegeben werden sollen.

In einer denkbaren Ausführungsform des erfindungsgemäßen Medizingeräts weist das Medizingerät eine Spracherkennungseinheit auf. Es ist auch hier unter "weist auf" zu verstehen, dass die Spracherkennungseinheit funktional Teil des Medizingeräts ist, aber zumindest teilweise auch außerhalb des Medizingeräts auf einem Server oder in einer Cloud realisiert sein kann. Unter Spracherkennungseinheit wird hier eine Funktionseinheit bezeichnet, die eine akustische Eingabe in einer natürlichen Sprache durch eine Spracherkennung in einen Text umsetzt, beispielsweise in einen Stream oder eine Datei mit Text-Zeichen, beispielsweise in ACII oder Unicode-Codierung. Üblicherweise wird eine Spracherkennung durch ein neuronales Netzwerk oder ein Hidden-Markov-Modell realisiert. Beispiele für derartige Spracherkennung in der Cloud sind beispielsweise Alexa oder Siri. Damit ist auch eine Kommunikation mit gesprochener Spracheeingabe über das Sprachsynthesesystem mit dem Patienten in Echtzeit möglich.

Auf vorteilhafte Weise ermöglicht eine Spracherkennungseinheit ein schnelles Erfassen von an den Patienten auszugebenden Informationen, insbesondere wenn die Sprache einer Bedienperson aufgenommen werden soll, die parallel das Medizingerät bedient.

In einer möglichen Ausführungsform des erfindungsgemäßen Medizingeräts weist das Medizingerät ein Large-Language-Modell auf. Darunter ist zu verstehen, dass zumindest funktional eine Implementierung eines Large-Language-Models in die Generierung eines Textes für die Sprachausgabeeinheit eingebunden ist. Dazu kann die Inferenz des Large-Language-Modells durch einen Prozessor oder einen speziellen Chip des Medizingeräts erfolgen, oder auch angebunden über eine Datenfernübertragung in einer Cloud oder einem Server. Das Large-Language-Modell ist ausgebildet, einen Ausgabetext für die Sprachausgabe an den Patienten zu generieren. Vorzugsweise ist dazu das Large-Language-Model mit dem erfindungsgemäßen Verfahren trainiert. Insbesondere ist es denkbar, ein generelles Large-Language-Modell, wie es beispielsweise von OpenAI oder anderen Firmen oder Open-Source-Projekten bereitgestellt wird, für den Kontext der Untersuchungen bzw. Behandlungen angepasst wird bzw. ein "Retraining" ausgeführt wird. Denkbar ist es auch, dass das Large-Language-Modell eine Übersetzung des Textes in eine andere Sprache vornimmt.

In einer denkbaren Ausführungsform des erfindungsgemäßen Medizingeräts ist das Large-Language-Modell ausgelegt, den Ausgabetext in Abhängigkeit von einem Status in einem Workflow des Magnetresonanzsystems zu erzeugen. Beispielsweise kann ein Prompt für das Large-Language-Modell zum Teil durch einen Status und/oder zum Teil durch eine Nutzereingabe bestehen.

Auf vorteilhafte Weise kann das Large-Language-Modell ähnlich einer menschlichen Bedienperson flexibel auf ungewöhnliche Situationen regieren und dem Patienten entsprechende Informationen ausgeben. Durch die Möglichkeit der Übersetzung des Textes bzw. Generieren des Ausgabetextes in einer anderen Sprache sind auch die Behandlung von Patienten mit unterschiedlichen Sprachen möglich oder auch sprachgrenzübergreifende Remote-Dienste.

Diese Vorteile der Vorrichtung gelten in gleicher Weise für das erfindungsgemäße Verfahren zur Durchführung der Behandlung bzw. Untersuchung.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die im Zusammenhang mit den Zeichnungen näher erläutert werden.

Es zeigen:
- Fig. 1: eine schematische Darstellung einer Ausführungsform eines erfindungsgemäßen Medizingeräts;
- Fig. 2: eine schematische Darstellung einer Ausführungsform eines erfindungsgemäßen Medizingeräts;
- Fig. 3: einen schematischen Ablaufplan eines erfindungsgemäßen Verfahrens zum Betrieb des erfindungsgemäßen Medizingeräts;
- Fig. 4: einen schematischen Ablaufplan eines erfindungsgemäßen Verfahrens zum Bereitstellen von Trainingsdaten für ein Large-Language-Modell eines Medizingeräts;
- Fig. 5: einen schematischen Ablaufplan eines erfindungsgemäßen Verfahrens zum Training des Sprachsynthesesystems eines erfindungsgemäßen Medizingeräts.

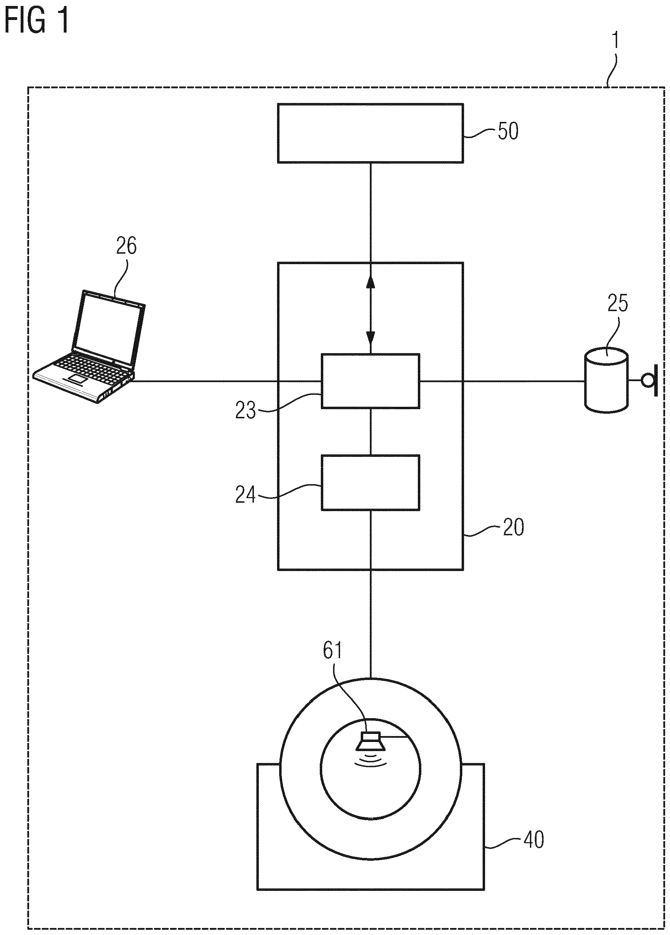

Fig. 1 zeigt eine schematische Darstellung einer Ausführungsform des erfindungsgemäßen Medizingeräts.

Das Medizingerät 1 der Fig. 1 zeigt bespielhaft einen Magnetresonanztomographen, bei dem Daten für eine Abbildung des Patienten 100 in einer Patienteneinheit 40 erfasst werden, die bei einem Magnetresonanztomographen einen Feldmagneten zum Erzeugen eines homogenen statischen Magnetfeldes, Gradientenspulen zum Erzeugen von Gradientenfeldern sowie einer Hochfrequenzeinheit zum Aussenden eines Anregungspulses und Empfangen von Magnetresonanzsignalen aufweist.

Zur Abbildung eines Untersuchungsobjektes werden Kernspins des Patienten 100 mit einem mit dem Magnetfeld des Feldmagneten ausgerichtet und durch ein magnetisches Wechselfeld des Anregungspulses zur Präzession um diese Ausrichtung anregen. Die Präzession bzw. Rückkehr der Spins aus diesem angeregten in einen Zustand mit geringerer Energie wiederum erzeugt als Antwort ein magnetisches Wechselfeld, das über Antennen als Magnetresonanzsignal von der Hochfrequenzeinheit empfangen wird.

Mit Hilfe von magnetischen Gradientenfeldern der Gradientenspulen wird den Signalen eine Ortskodierung aufgeprägt, die nachfolgend eine Zuordnung von dem empfangenen Signal zu einem Volumenelement ermöglicht. Das empfangene Signal wird dann ausgewertet und eine dreidimensionale bildgebende Darstellung des Untersuchungsobjektes bereitgestellt.

Die Patienteneinheit 40 wird von einer Steuereinheit 20 bei der Bilderfassung gesteuert, die eine Steuerung 23 bzw. einen Prozessor aufweist. Die Steuerung 23 koordiniert die Signale der Gradientenspulen und des Anregungspulses sowie den Empfang der Magnetresonanzsignale einer Bilderfassung in einer sogenannten Sequenz.

Die Kommunikation mit dem Patienten 100 erfolgt üblicherweise auf akustischem Wege durch Sprachmitteilungen, die dem Patienten 100 über einen Lautsprecher 61 oder Kopfhörer in der Patienteneinheit 40 zugespielt werden. Um Störungen durch Magneten des Kopfhörers oder Lautsprechers 61 zu vermeiden, kann der elektro-akustische Wandler auch außerhalb des Feldmagneten angeordnet sein und der Schall über einen Schallleiter wie einen Schlauch zugeführt werden. Im Stand der Technik wird hierbei üblicherweise eine Spracheingabe eines Nutzers über ein Mikrofon einer Gegensprechanlage analog oder in digitalisierter Form zu dem Lautsprecher 61 übertragen und dort ausgegeben.

Das erfindungsgemäße Medizingerät 1 hingegen weist eine Sprachsynthesesystem 24 bzw. eine Sprachsyntheseeinheit auf, die die akustische Sprache zur Ausgabe über den Lautsprecher 61 erzeugt. Das Sprachsynthesesystem 24 kann beispielsweise in der Steuereinheit 20 angeordnet und mit der Steuerung 23 in Signalverbindung sein. Es ist aber beispielsweise auch denkbar, das Sprachsynthesesystem 24 am Ort des Lautsprechers 61 angeordnet ist und mit der Steuerung 23 über eine kabelgebundene, Bluetooth- eine LAN- oder WLAN-Signalverbindung steht.

Das Sprachsynthesesystem 24 erhält die Information bzw. Anweisung zur Ausgabe an den Patienten dabei nicht in analoger oder digitaler Repräsentation der Töne wie beispielsweise als WAV-Datei zum Abspielen, sondern in einer textlichen bzw. semantischen Repräsentation, die den Inhalt der Information bzw. Anweisung wiedergibt, beispielsweise als Textstring oder im XML-Format. Das Sprachsynthesesystem 24 erzeugt daraus eine akustische Repräsentation zur Ausgabe über den Lautsprecher 61, indem beispielsweise gespeicherte Phoneme aneinandergefügt werden. Denkbar für das Sprachsynthesesystem 24 sind auch andere Ansätze, die beispielsweise neuronale Netzwerke zur Generierung der Sprachausgabe oder auch nur einzelner Phoneme nutzen.

Das Sprachsynthesesystem 24 steht vorzugsweise mit der Steuerung 23 in Signalverbindung und erhält von dieser die Ausgaben, beispielsweise in Textform als String, Stream von Zeichen oder Datei. Die Steuerung kann diese beispielsweise über eine Bedienschnittstelle 26 wie ein Terminal direkt vom Benutzer erhalten. Denkbar ist, dass dieser die Texte eintippt oder aus einer Auswahl vorgefertigter Texte auswählt. Es ist aber auch möglich, dass das Medizingerät 1 eine Spracherkennungseinheit 25 aufweist, über die der Bediener die Information bzw. Anweisung in natürlicher Sprache über ein Mikrofon eingibt. Die Spracherkennungseinheit 25 wandelt diese in Textform um, die dann von der Steuerung 23 oder direkt an das Sprachsynthesesystem 24 übermittelt wird.

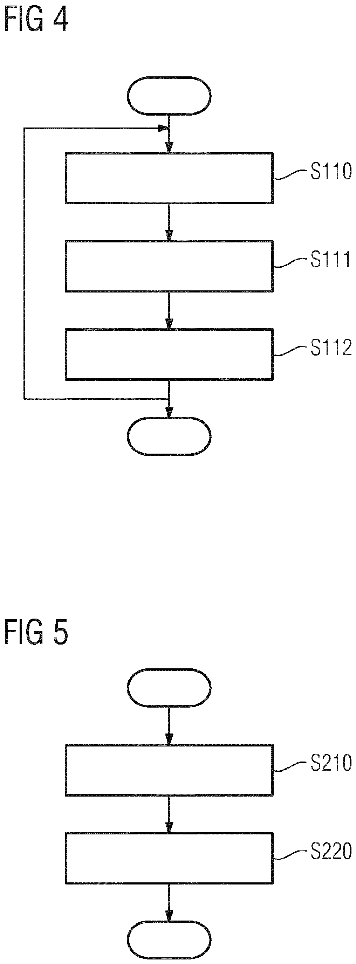

Denkbar ist es auch, dass bei der Generierung ein Large-Language-Modell (LLM) 50 zum Einsatz kommt. Das LLM 50 kann dann die Information oder Anweisung zur Ausgabe generieren. Als Input bzw. Teil eines Prompts kann dem LLM 50 beispielsweise ein Status der Bilderfassung bzw. Behandlung von der Steuerung 23 erhalten, eine Eingabe des Bedieners, und/oder eine Frage des Patienten 100 zugeführt werden, sodass die erzeugte Information situationsbezogen ist. Das LLM 50 kann dann das Ergebnis direkt oder über die Steuerung 23 an das Sprachsynthesesystem 24 zur Ausgabe übermitteln. Das LLM 50 wird vorzugsweise mit Trainingsdaten, die mit dem nachfolgend zu Fig. 4 näher erläuterten Verfahren gewonnen wurden, trainiert bzw. angepasst, um die Ausgaben spezifisch an den Anwendungszweck in dem Medizingerät 1 anzupassen.

Denkbar ist es auch, dass das LLM 50 zur Übersetzung einer Eingabe in eine andere Sprache genutzt wird. Dabei kann das LLM 50 einen über die Bedienschnittstelle 26 oder die Spracherkennungseinheit 25 erfassten Text in einen Text in einer Zielsprache übersetzen, der dann wiederum durch die Sprachsyntheseeinheit 24 ausgegeben wird. Dabei ist es erforderlich, dass die Sprachsyntheseeinheit 24 die entsprechende Zielsprache auch beherrscht bzw. durch entsprechendes Training oder Versorgen mit entsprechenden Parametern bzw. Daten erlernen kann.

Auf vorteilhafte Weise können so auch Patienten mit unterschiedlichen Sprachen durch Bedienpersonen untersucht bzw. behandelt werden, ohne dass Bedienpersonal mit entsprechenden Sprachkenntnissen verfügbar sein muss.

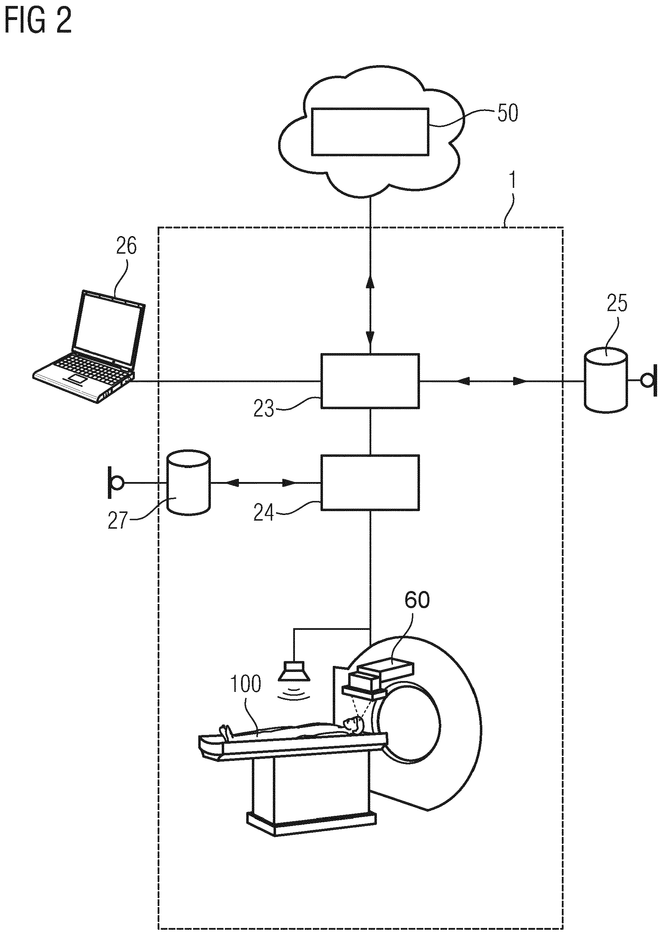

In Fig. 2 ist eine andere denkbare Ausführungsform eines erfindungsgemäßen Medizingeräts 1 schematisch dargestellt. Die Ausführungsform der Fig. 2 unterscheidet sich zum einen dadurch, dass anstelle der Patienteneinheit 40 eine Therapieeinheit 60 tritt. Die Therapieeinheit 60 kann beispielsweise, wie schematisch angedeutet, eine Bestrahlungseinheit mit einem LINAC aufweisen.

Zum anderen ist in der Ausführungsform der Fig. 2 das LLM 50 nicht physisch in das Medizingerät 1 integriert, sondern in einem Server oder eine Cloudinfrastruktur realisiert und logisch in das Medizingerät 1 eingebunden. In gleicher Weise ist hier die Spracherkennungseinheit 25 physisch ausgegliedert und nur logisch über eine Datenfernverbindung angegliedert. Die Sprache der Bedienperson kann hier über ein Mikrofon erfasst, digitalisiert und an die Spracherkennungseinheit 25 übertragen werden und der transskribierte Text in umgekehrter Richtung übertragen werden. Gleiches ist für einen Propmt an das LLM 50 und die generierte Antwort denkbar. Die Erfindung betrifft hier die logische bzw. funktionale Einbindung von LLM 50 und Spracherkennungseinheit 25 und ist nicht beschränkt auf eine spezifische physische Realisierung.

Weiterhin weist die Ausführungsform der Fig. 2 eine Programmierschnittstelle 27 für das Sprachsynthesesystem auf. Die Programmierschnittstelle 27 ist dazu ausgebildet, die akustische Sprache und/oder Stimme, die von dem Sprachsynthesesystem 24 aus einem zugeführten Text erzeugt wird, zu verändern. Denkbar ist es beispielsweise, dass über die Programmierschnittstelle 27 dem Sprachsynthesesystem 24 ein Satz neuer Phoneme zugeführt wird, anhand dessen die akustische Sprachausgabe erzeugt wird. Bei einer parametrisierten synthetischen Stimme kann es sich um einen veränderten Parametersatz handeln. Bei einer Realisierung mittels eines neuronalen Netztes kann es sich um einen Satz entsprechender Gewichtungsfaktoren für die Verbindung der Knoten handeln.

In einer Ausführungsform der Programmierschnittstelle 27 ist diese insbesondere ausgebildet, aus Spracheingaben bzw. Sprachproben einer Zielperson über ein Mikrofon die entsprechenden Parametersätze, d.h. Phoneme oder Parameter für die synthetische Stimme bzw. das neuronale Netzwerk für das Sprachsynthesesystem 24 zu erzeugen, sodass nach der Übertragung der Parametersätze das Sprachsynthesesystem 24 Sprachausgaben mit der Stimme der Zielperson erzeugt. Wie bereits zu LLM 50 und Spracherkennungseinheit 25 erläutert, kann die Programmierschnittstelle 27 für das Sprachsynthesesystem 24 auch in einer Cloud realisiert sein, insbesondere komplexere Funktionen wie das Trainiern bzw. Anpassen eines Parametersatzes für ein neuronales Netzwerk. Die Sprachproben können beispielsweise auch mittels eines Smart-Phones erfasst werden und dem Sprachsynthesesystem 24 als Tondatei zugeführt werden.

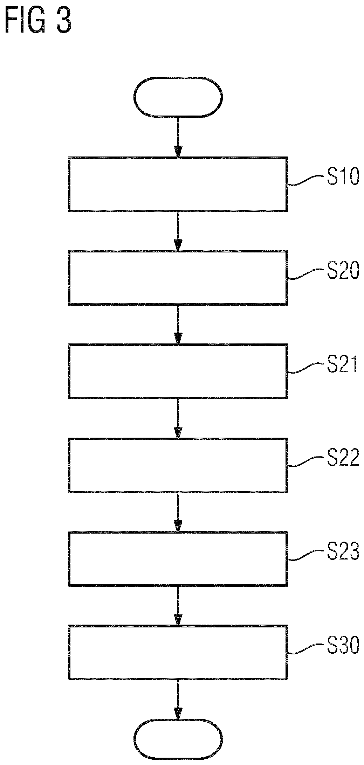

Fig. 3 zeigt einen schematischen Ablaufplan eines erfindungsgemäßen Verfahrens zum Betrieb des erfindungsgemäßen Medizingeräts 1. In einem Schritt S10 des Verfahrens wird der Patient 100 positioniert. Darunter ist zu verstehen, dass der Patient 100 in eine Lage relativ zu dem Medizingerät 1 gebracht wird, die für die Untersuchung bzw. Behandlung erforderlich ist. Bei einem Magnetresonanztomographen befindet sich der Patient 100 üblicherweise auf einem Patiententisch in der Patienteneinheit 40 bzw. der Therapieeinheit 60. Um die Bedienperson vor Strahlung zu schützen bzw. um Störungen der Bilderfassung zu vermindern, befindet sich die Bedienperson üblicherweise in einem anderen Raum und kommuniziert im Stand der Technik mit dem Patienten 100 über elektronische Mittel, beispielsweise über Mikrofon und Lautsprecher, wobei die Sprache der Bedienperson direkt in Echtzeit übertragen wird.

In einem weiteren Schritt des erfindungsgemäßen Verfahrens hingegen wird in einem Schritt S20 eine Sprachanweisung bzw. Information in Sprachform an den Patienten 100 mittels des Sprachsynthesesystems 24 ausgegeben. Mit anderen Worten, das Sprachsynthesesystem 24 empfängt die Anweisung in Textform, also nicht in einer analogen oder digitalen Form der akustischen Sprache. Das Sprachsynthesesystem 24 wandelt die Textform in eine akustische Ausgabe in natürlicher Sprache, die über z.B. einen Lautsprecher 61 oder anderen akustischen Wandler ausgegeben wird. Dabei ist es denkbar, dass die akustische Ausgabe des Sprachsynthesesystems 24 zunächst noch digitalisiert und komprimiert vorliegt und vor der Ausgabe noch dekomprimiert, gewandelt und/oder verstärkt wird.

Auf vorteilhafte Weise ermöglicht das Sprachsynthesesystem 24, auch bei Wechsel der Bedienperson oder sogar in Abwesenheit eine gewohnte Stimme für den Patienten 100 bereitzustellen, die ihn beruhigt und auch die Verständlichkeit verbessern kann.

Die Information bzw. Anweisung kann dabei unterschiedliche Ursprünge bzw. Quellen haben. Es ist eine denkbare Möglichkeit, dass die Bedienperson in einem Schritt S21 die Anweisung in Textform an einer Bedienschnittstelle 26 auswählt oder eingibt. Die Steuerung 23 übermittelt dann die Anweisung in Textform an das Sprachsynthesesystem 24 zur Ausgabe.

Es ist auch denkbar, dass eine Spracherkennungseinheit 25 in einem Schritt S22 eine Anweisung oder Information in natürlicher Sprache von einer Bedienperson erfasst und in Textform an die Steuerung 23 oder direkt an das Sprachsynthesesystem 24 weiterreicht.

Es ist auch möglich, dass die Information oder Anweisung in Textform ganz oder teilweise in einem Schritt S23 von einem Large-Language-Model (LLM) 50 erzeugt wird. Dabei kann beispielsweise eine Frage des Patienten 100 von einer Spracherkennungseinheit 25 in Textform gebracht werden und dem LLM 50 als Teil eines Prompts zugeführt werden, um eine Antwort auf die Frage zu bestimmen, die dann von dem Sprachsynthesesystem 25 ausgegeben zu werden.

Ebenso ist es denkbar, dass ein Systemzustand des Medizingeräts in den Prompt eingeht, der beispielsweise von der Steuerung 23 dem LLM 50 zugeführt wird oder beim Generieren des Prompts genutzt wird. Es wäre auch denkbar, dass das LLM 50 erforderliche Informationen, wie beispielsweise die verbleibende Dauer der Untersuchung bzw. der Behandlung vom System abfragt.

In einem weiteren Schritt S30 wird ein Untersuchungsschritt oder Behandlungsschritt bzw. Teilschritt davon an dem Patienten 100 ausgeführt. Dabei ist es denkbar, dass die Anweisung bzw. Information von dem Sprachsynthesesystem 24 in dem Schritt S20 vorher, nachher oder auch währenddessen ausgegeben wird, beispielsweise um den Patienten 100 anzuweisen, während einer Aufnahme oder Bestrahlung den Atem anzuhalten.

Fig. 4 zeigt einen schematischen Ablaufplan eines erfindungsgemäßen Verfahrens zum Bereitstellen von Trainingsdaten für ein Large-Language-Modell eines Medizingeräts 1. Als Trainingsdaten werden die unterschiedlichen Eingaben, Ausgaben und Parameter während eines Betriebes des Medizingeräts 1 erfasst, wobei vorzugsweise die Informationen und Anweisungen von einer Bedienperson gesprochen bzw. eingegeben werden.

Dazu wird in einem Schritt S110 eine Behandlung oder Untersuchung an einem Patienten mit dem Medizingerät durchgeführt.

Währenddessen werden in einem Schritt S111 Sprachanweisungen bzw. Informationen an den Patienten 100 erfasst. Es kann sich dabei um Sprachanweisungen bzw. Informationen handeln, die eine Bedienperson akustisch gibt, über ein Bedienschnittstelle 26 eingibt oder auswählt, oder auch von der Steuerung 23 generiert werden.

Parallel dazu werden von der Steuerung 23 in einem Schritt S112 Systemzustände des Medizingeräts 1 erfasst, um den jeweiligen Status der Untersuchung bzw. Behandlung zu dokumentieren.

Dabei wird eine zeitliche Relation der erfassten Daten festgehalten, beispielsweise durch Zeitstempel der Steuerung 23. Auf diese Weise ist es möglich, die zeitlichen und kausale Zusammenhänge der unterschiedlichen erfassten Daten festzuhalten,

Vorzugsweise werden die Schritte S111 und S112 wiederholt, beispielsweise bei einer Vielzahl von Untersuchungen bzw. Behandlungen. So können beispielsweise patientenspezifische Abweichungen und andere Änderungen erfasst und von der LLM 50 nach dem Training berücksichtigt werden.

In Fig. 5 ist schließlich ein Verfahren zum Training des Sprachsynthesesystems eines erfindungsgemäßen Medizingeräts schematisch dargestellt. Dazu werden in einem Schritt S210 Sprachproben einer Zielperson erfasst, wobei mit Zielperson die Person bezeichnet ist, deren Stimme das Sprachsynthesesystem 24 durch das Training nachahmen soll. Die Art der Daten hängt dabei von der Art des Sprachsynthesesystems 24 ab. Ist dieses beispielsweise Phonembasiert, werden vorzugsweise ein möglichst vollständiger Satz an Phonemen erfasst, beispielsweise durch Vorlesen und Aufnehmen eines Textes mit möglichst vielen unterschiedlichen Phonemen. Bei einer künstlichen Stimme, die vor allem auf akustischen Parametern wie Grundfrequenzen, Resonanzen, Oberwellen und An- und Abklingverhalten beruht, werden entsprechende Parameter in den Sprachproben erfasst. Für ein Sprachsynthesesystem 24 beruhend auf neuronalen Netzen wird versucht, für möglichst viele unterschiedliche Texte Sprachproben zu erfassen. Denkbar ist auch ein zweistufiges Sprachsynthesesystem 24, wobei eine erste Stufe für eine Umsetzung von Text in Phoneme ausführt und nachfolgend eine zweite Stufe für eine akustische Ausgabe der Phoneme sorgt. Eine oder beide der Stufen können durch neuronale Netze realisiert sein. Um beliebige Stimmen nachzuahmen ist dabei vor allem die zweite Stufe relevant.

In einem weiteren Schritt S220 wird das Sprachsynthesesystems 24 mit den Sprachproben trainiert. Das Training ist wieder abhängig von der Realisierung des Sprachsynthesesystems 24. Basiert es beispielsweise auf Phonemen als Basis für die akustische Ausgabe, kann eine neue Stimme durch Austausch der gespeicherten Phoneme mit den neu erfassten erfolgen. Bei einer parameterbasierten synthetischen Stimmerzeugung werden die Sprachproben auf die entsprechenden Parameter analysiert und diese in dem Sprachsynthesesystem 24 gespeichert. In einem Sprachsynthesesystem 24, das auf einem ein- oder mehrstufigen neuronalen Netzwerk basiert, wird das Netzwerk trainiert, indem die Netzwerkparameter mittels der üblichen Backpropagation angepasst werden. Dabei wird das Ziel verfolgt, die vom Netzwerk erzeugten Ausgabedaten so gut wie möglich an die tatsächlichen Sprachproben, die als Eingangsdaten gesammelt wurden, anzupassen. Das Training erfolgt dabei vorzugsweise wegen des erhöhten Bedarfs an Rechenleistung in einem Server oder Clouddienst, wobei anschließend die ermittelten Parameter des neuronalen Netzwerks in das Sprachsynthesesystem 24 übertragen werden.

Das trainierbare Sprachsynthesesystem 24 ermöglicht insbesondere bei Kindern oder dementen Patienten 100, diese durch gewohnte Stimmen zu beruhigen und die Untersuchung bzw. Behandlung erfolgreich durchzuführen.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Medizingerät, wobei das Medizingerät (1) ein Sprachsynthesesystem (24) aufweist, das ausgelegt ist, Informationen in natürlicher Sprache an einen Patienten (100) auszugeben.

2. Medizingerät nach Anspruch 1, wobei das Medizingerät (1) eines aus einer Gruppe von Magnetresonanztomograph, Computertomograph, PET-System oder Bestrahlungsvorrichtung ist.

3. Medizingerät nach Anspruch 1 oder 2, wobei das Sprachsynthesesystem (24) ausgebildet ist, unterschiedliche Stimmen für die Sprachausgabe zu erlernen und bei der Sprachausgabe zu nutzen.

4. Medizingerät nach einem der vorhergehenden Ansprüche, wobei das Sprachsynthesesystem (24) eine Textschnittstelle zur Eingabe der Sprachanweisungen aufweist.

5. Medizingerät nach Anspruch 3, wobei das Medizingerät (1) eine Spracherkennungseinheit (25) aufweist, die ausgebildet ist, aus einer Spracheingabe einen Text für eine Sprachausgabe zu generieren.

6. Medizingerät nach einem der vorhergehenden Ansprüche, wobei das Medizingerät (1) ein Large-Language-Modell (50) aufweist, wobei das Large-Language-Modell (50) ausgebildet ist, einen Ausgabetext für das Sprachsynthesesystem (24) zu generieren.

7. Medizingerät nach Anspruch 6, wobei das Large-Language-Modell (50) ausgelegt ist, den Ausgabetext in Abhängigkeit von einem Status in einem Workflow des Medizingeräts (1) zu erzeugen.

8. Verfahren zur Durchführung einer Behandlung oder Untersuchung mit einem Medizingerät (1) nach einem der Ansprüche 1 bis 7, wobei das Verfahren die Schritte aufweist:
- Positionieren (S10) eines Patienten (100);
- Ausgeben (S20) einer Sprachanweisung an den Patienten (100) mittels des Sprachsynthesesystems (24);
- Ausführen (S30) eines Untersuchungsschritts bzw. Behandlungsschritts an dem Patienten (100) mit dem Medizingerät (1).

9. Verfahren nach Anspruch 8 für ein Medizingerät (1) nach Anspruch 5, wobei eine Bedienperson in einem Schritt (S22) die Sprachanweisung akustisch über die Spracherkennungseinheit (25) eingibt.

10. Verfahren nach Anspruch 8 oder 9 für ein Medizingerät (1) nach Anspruch 6, wobei das Large-Language-Model (50) in einem Schritt (S23) die Sprachanweisung in Abhängigkeit von einem Systemzustand erzeugt.

11. Verfahren zum Bereitstellen von Trainingsdaten für ein Large-Language-Modell (50) eines Medizingeräts (1) nach Anspruch 6, wobei das Verfahren die Schritte aufweist:
- Durchführen einer Behandlung (S110) oder Untersuchung an einem Patienten (100) mit dem Medizingerät (1);
- Erfassen von Sprachanweisungen (S111) an den Patienten (100);
- Erfassen von Systemzuständen (S112) des Medizingeräts (1),
wobei eine zeitliche Relation der erfassten Daten festgehalten wird;
Wiederholen der Schritte an einem anderen Patienten und/oder mit einer anderen Bildgebungssequenz zur Bilderfassung.

12. Verfahren zum Training des Sprachsynthesesystems (25) des Medizingeräts (1) nach Anspruch 3, wobei das Verfahren die Schritte aufweist:
- Erfassen von Sprachproben (S210) einer Zielperson;
- Trainieren des Sprachsynthesesystems (S220) mit den Sprachproben.
